(19) Europäisches Patentamt / European Patent Office / Office européen des brevets

(11) **EP 2 630 105 B1**

(12) **EUROPEAN PATENT SPECIFICATION**

(45) Date of publication and mention
of the grant of the patent:
**18.04.2018 Bulletin 2018/16**

(51) Int Cl.:
***C07C 2/36*** *(2006.01)*    ***C07C 11/02*** *(2006.01)*
***B01J 31/14*** *(2006.01)*

(21) Application number: **11833662.7**

(22) Date of filing: **28.09.2011**

(86) International application number:
**PCT/CA2011/001086**

(87) International publication number:
**WO 2012/051698 (26.04.2012 Gazette 2012/17)**

(54) **ETHYLENE OLIGOMERIZATION**

ETHYLENOLIGOMERISIERUNG

OLIGOMÉRISATION D'ÉTHYLÈNE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.10.2010 CA 2718455**

(43) Date of publication of application:
**28.08.2013 Bulletin 2013/35**

(73) Proprietor: **Nova Chemicals (International) S.A.
1700 Fribourg (CH)**

(72) Inventors:
• **CARTER, Charles Ashton Garret
Calgary
Alberta T3A 6L7 (CA)**
• **CHISHOLM, P., Scott
Calgary
Alberta T2E 7K7 (CA)**
• **BROWN, Stephen John
Calgary
Alberta T2Z 2G7 (CA)**
• **JABER, Isam
Calgary
Alberta T2E 4T5 (CA)**

(74) Representative: **Watson, Robert James et al
Mewburn Ellis LLP
City Tower
40 Basinghall Street
London EC2V 5DE (GB)**

(56) References cited:
**WO-A1-2010/034101**    **US-A- 5 066 631**
**US-A1- 2010 222 622**    **US-B2- 7 378 537**

**Description**

## TECHNICAL FIELD

This invention relates to the oligomerization of ethylene using a chromium/diphosphine catalyst and a methylaluminoxane activator.

## BACKGROUND ART

Aluminoxanes are commercially available items that are used as activators for olefin polymerization catalysts. Methylaluminoxane (or "MAO") is commonly used because it generally provides high catalyst activity.

MAO can be prepared by the partial hydrolysis of trimethylaluminum ("TMA") using a number of methods that are well known to those skilled in the art. The literature documents several difficulties with the synthesis, storage and transportation of MAO. Most notably, solutions of MAO in hydrocarbon solvents are known to "gel" and/or form solid precipitates after preparation and storage.

The patent literature describes two general approaches to mitigate these problems. One approach to improve the solubility of MAO solutions is to include a longer chain aluminum alkyl in the aluminoxane synthesis (or, alternatively, to add the longer chain aluminum alkyl after synthesis of the MAO). U.S. Patent 5,066,631 exemplifies the use of tri-n-octyl aluminum for this purpose. The resulting product is generally referred to as "modified" MAO (or "MMAO") to indicate the presence of the longer chain alkyl groups. Modified MAO is soluble in aliphatic solvents such as hexane, octene and cyclohexane. MMAO is commercially available in aliphatic solvents (such as the product sold under the trade name MMAO-3 by Akzo Nobel).

Another approach to mitigate the solubility/gel problems is to use an aromatic solvent because "pure" MAO" is generally more soluble in aromatic solvents than aliphatic solvents. "Pure" MAO (i.e. MAO which does not contain higher alkyl groups) is commercially available as a toluene solution from Akzo Nobel. WO 2010/034101 discloses such process for the oligomerisation of ethylene in the presence of methylaluminoxane in a toluene solution. However, as noted in U.S. 5,066,631, aliphatics are "preferred catalyst solvents because they are less toxic than aromatic hydrocarbons".

Commercially available MMAO and MAO are both excellent activators for catalysts used in ethylene polymerization.

More recently, the use of aluminoxanes as activators for catalysts to selectively oligomerize ethylene has recently been proposed. One particular family of selective oligomerization catalysts is prepared by contacting chromium with a bridged diphosphine ligand. We have observed a difference between commercially available MMAO and MAO when used with these oligomerization catalysts. Specifically, MMAO (in aliphatic solvent) has been observed to produce large amounts of by product polymer whereas regular MAO (in toluene) was observed to produce less polymer. It is not presently known whether the amount of by product polymer being formed is influenced by the type of solvent (aromatic versus non-aromatic), the type of alkyl aluminum used to prepare the aluminoxane, some combination of the two or none of the above.

However, the use of aromatic solvents for the aluminoxane can be undesirable - from the perspective of potential toxicological issues and from the perspective of the need to separate the aromatic solvent from the final products.

We have now discovered that a useful activator for ethylene oligomerization may be prepared by the partial hydrolysis of TMA in a non-aromatic solvent.

## DISCLOSURE OF THE INVENTION

The present invention provides a process for the oligomerization of ethylene, said process comprising:

A) a first step wherein an activator is prepared by the partial hydrolysis of a solution of an alkyl aluminum with water;
B) a second step comprising contacting:

a) said activator;
b) a catalyst comprising a source of chromium and a ligand defined by the formula $(R^1)(R^2)\text{-}P^1\text{-bridge-}P^2(R^3)(R^4)$ wherein $R^1$, $R^2$, $R^3$ and $R^4$ are independently selected from the group consisting of hydrocarbyl and heterohydrocarbyl and the bridge is a divalent moiety that is bonded to both phosphorus atoms; and
c) ethylene,

wherein said a), b) and c) are contacted in an oligomerization reactor under oligomerization conditions, with the provisos that:

1) said first step is conducted in a non aromatic solvent for said alkyl aluminum;

2) said alkyl aluminum consists of trimethylaluminum;

3) the amount of said aluminum used is said first step is from 0.5 to 3 weight %, based on the combined weight of said aluminum and said non aromatic solvent; and

4) said activator is used in said second step within 7 days from when said activator is prepared.

## BEST MODE FOR CARRYING OUT THE INVENTION

## PART A CATALYST SYSTEM

The catalyst system used in the process of the present invention must contain three essential components, namely:

(i) a source of chromium:

(ii) a defined diphosphine ligand; and

(iii) an activator.

Preferred forms of each of these components are discussed below, starting with the activator (iii).

Activator (iii)

The activator that is used in the process of this invention is characterized by a number of features, including:

1) it is prepared using trimethylaluminum as the only alkyl aluminum (i.e. not a higher alkyl aluminum or a mixture of alkyl aluminums);

2) it is prepared by partial hydrolysis;

3) the partial hydrolysis is conducted under dilute conditions;

4) the partial hydrolysis is conducted in a non aromatic solvent; and

5) the activator is used within 7 days of being prepared.

Further discussions of these characteristics follows.

Firstly, the alkyl aluminum "consists of" trimethylaluminum. The term "consists of" is meant to convey its normal meaning - i.e. to exclude the use of other alkyl aluminums. As shown in the examples, the use of a commercially available aluminoxane that is prepared with both trimethylaluminum and higher alkyl aluminums (such as tributylaluminum or trioctylaluminum) leads to undesirably high levels of by polymeric by product.

The activator is prepared by the partial hydrolysis of TMA with water. As used herein, the term "partial hydrolysis" means that the amount of water that is provided is less than the minimum amount that would be required to react with all methyl groups contained in the TMA (on a stoichiometric basis). For clarity, one mole of $H_2O$ may theoretically react with two methyl groups. Thus, the minimum amount of water required for a (theoretical) "full hydrolysis" of TMA would be 1.5 moles of water per mole of TMA. The preferred water/TMA molar ratios are from 0.3/1 to 1/1.

The water may be provided in the form of liquid water (that has preferably been distilled and deionized) or in the form of water that is associated with a solid particulate carrier. Examples of solid particulate carriers with associated water include metal salts having water of hydration (such as copper sulfate or $CuSO_4 \cdot XH_2O$) and metal oxides such as silica having physically associated water. The use of such solid particulate carriers in the preparation of aluminoxanes is well known to those skilled in the art.

In one embodiment, the water is added directly to a solution of TMA.

In another embodiment, a solution of water in the non-aromatic solvent is initially prepared and this solution is then added to the solution of TMA. It will be recognized by those skilled in the art that the solubility of water in non-aromatic hydrocarbons is extremely low. The use of a co-solvent is contemplated to mitigate this problem - where the term "co-solvent" refers to a liquid that is soluble in the non-aromatic solvent, with the further requirement that water is more soluble in the co-solvent than the non-aromatic solvent. Examples include alcohols and ethers such as methanol, ethanol, isopropanol, tertiary butyl alcohol and methyl tertiary butyl ether.

For clarity, the present invention does not encompass aluminoxanes that are prepared solely by thermolysis. The use of thermolysis to prepare aluminoxanes is widely disclosed in the patent literature.

The activator must be prepared in a non aromatic solvent. It will be recognized by those skilled in the art that aromatic solvents (such as benzene, toluene and xylene) are excellent solvents for MAO and may generally be used to prepare high active MAO solutions. However, the use of aromatic solvents is not encompassed by the present invention for the reasons previously noted.

Preferred solvents include $C_6$ to $C_{20}$ aliphatic hydrocarbons; $C_6$ to $C_{20}$ olefins and mixtures thereof. Examples of preferred aliphatic hydrocarbons include hexanes, heptanes and octanes. These hydrocarbons may be linear, branched or cyclic (such as cyclohexane). Examples of $C_6$ to $C_{20}$ olefins include hexenes, heptenes, octenes etc. Likewise, these octenes may be linear or branched and the unsaturation may be at the alpha or an internal position. A mixture of hexene and octene (which may be prepared by the present process) is a particularly preferred non aromatic solvent.

Care should be taken when contacting the water with the TMA solution because the hydrolysis of TMA can be explosively violent.

The process of this invention requires that the partial hydrolysis of TMA is conducted under dilute conditions - specifically that the concentration of aluminum is less than 3 weight % based on the weight of the aluminum plus the weight of the non aromatic solvent. For the avoidance of doubt, the concentration of aluminum is calculated as follows:

$$\text{Weight \% Al} = \frac{\text{weight Al}}{\text{weight Al + weight solvent}} \times 100\%$$

The partial hydrolysis may be undertaken at room temperature. Alternatively, the initial contact of water with TMA may occur at low temperature (as shown in the examples). It is generally preferred to conduct the partial hydrolysis in a stirred reactor.

For the purpose of this invention, the activator may be defined as being "prepared" after 5 minutes has elapsed from the initial contact of water with the TMA solution (although, for avoidance of doubt, it may also be noted that the reaction between the water and TMA might not be complete within the 5 minutes). The "prepared" activator must then be used in the subsequent oligomerization reaction within 7 days. Activator that is older than 7 days may form precipitates that render it unsuitable for use in the present invention. The examples illustrate the preparation of the activator in a batch process. The use of a semi batch process or a continuous process is also contemplated by the present invention. A continuous process could be undertaken by directing the flows of trimethylaluminum, solvent and water to a continuously operated reactor and adjusting the flow rates to match the desired reaction/preparation time for the activator using techniques that are well known to those skilled in the art. The literature provides teachings that suggest that optimum reaction time for MAO formation can be in the range of several hours (in contrast to the above definition of 5 minutes as the time for the activator to be "prepared" - the 5 minutes is arbitrary).

The oligomerization step of the present invention is conducted by contacting the above described activator with a catalyst comprising a source of chromium and a bridged diphosphine ligand and these components are described in further detail below.

Chromium Source ("Component (i)")

Any source of chromium which allows the oligomerization process of the present invention to proceed may be used. Preferred chromium sources include chromium trichloride; chromium (III) 2-ethylhexanoate; chromium (III) acetylacetonate and chromium carboxyl complexes such as chromium hexacarboxyl.

Ligand Used in the Oligomerization Process ("Component (ii)")

In general, the ligand used in the oligomerization process of this invention is defined by the formula $(R^1)(R^2)\text{-}P^1\text{-bridge-}P^2(R^3)(R^4)$ wherein $R^1$, $R^2$, $R^3$ and $R^4$ are independently selected from the group consisting of hydrocarbyl and heterohydrocarbyl and the bridge is a divalent moiety that is bonded to both phosphorus atoms.

The term hydrocarbyl as used herein is intended to convey its conventional meaning - i.e. a moiety that contains only carbon and hydrogen atoms. The hydrocarbyl moiety may be a straight chain; it may be branched (and it will be recognized by those skilled in the art that branched groups are sometimes referred to as "substituted"); it may be saturated or contain unsaturation and it may be cyclic. Preferred hydrocarbyl groups contain from 1 to 20 carbon atoms. Aromatic groups - especially phenyl groups - are especially preferred. The phenyl may be unsubstituted (i.e. a simple $C_6H_5$ moiety) or contain substituents, particularly at an ortho (or "o") position.

Similarly, the term heterohydrocarbyl as used herein is intended to convey its conventional meaning - more particularly, a moiety that contains carbon, hydrogen and heteroatoms (such as O, N, R and S). The heterocarbyl groups may be straight chain, branched or cyclic structures. They may be saturated or contain unsaturation. Preferred heterohydrocarbyl groups contain a total of from 2 to 20 carbon + heteroatoms (for clarity, a hypothetical group that contains 2 carbon atoms and one nitrogen atom has a total of 3 carbon + heteroatoms).

It is preferred that each of $R^1$, $R^2$, $R^3$ and $R^4$ is a phenyl group (with an optional substituent in an ortho position on one or more of the phenyl groups).

Highly preferred ligands are those in which $R^1$ to $R^4$ are independently selected from the group consisting of phenyl, *o*-methylphenyl (i.e. ortho-methylphenyl), *o*-ethylphenyl, *o*-isopropylphenyl and *o*-fluorophenyl. It is especially preferred that none of $R^1$ to $R^4$ contains a polar substituent in an ortho position. The resulting ligands are useful for the selective tetramerization of ethylene to octene-1 with some co product hexene also being produced. The term "bridge" as used herein with respect to the ligand refers to a divalent moiety that is bonded to both of the phosphorus atoms in the ligand - in other words, the "bridge" forms a link between $P^1$ and $P^2$. Suitable groups for the bridge include hydrocarbyl and an inorganic moiety selected from the group consisting of $N(CH_3)-N(CH_3)-$, $-B(R^6)-$, $-Si(R^6)_2-$, $-P(R^6)-$ or $-N(R^6)-$ where $R^6$ is selected from the group consisting of hydrogen, hydrocarbyl and halogen.

It is especially preferred that the bridge is $-N(R^5)-$ wherein $R^5$ is selected from the group consisting of hydrogen, alkyl, substituted alkyl, aryl, substituted aryl, aryloxy, substituted aryloxy, halogen, alkoxycarbonyl, carbonyloxy, alkoxy, aminocarbonyl, carbonylamino, dialkylamino, silyl groups or derivatives thereof and an aryl group substituted with any of these substituents. A highly preferred bridge is amino isopropyl (i.e. when $R^5$ is isopropyl).

## PART B PROCESS CONDITIONS

The chromium (component (i)) and ligand (component (ii)) may be present in any molar ratio which produces oligomer, preferably between 100:1 and 1:100, and most preferably from 10:1 to 1:10, particularly 3:1 to 1:3. Generally the amounts of (i) and (ii) are approximately equal, especially 1/1 and 1/2.

Components (i)-(iii) of the catalyst system utilized in the present invention may be added together simultaneously or sequentially, in any order, and in the presence or absence of ethylene in any suitable solvent, so as to give an active catalyst. For example, components (i), (ii) and (iii) and ethylene may be contacted together simultaneously, or components (i), (ii) and (iii) may be added together simultaneously or sequentially in any order and then contacted with ethylene, or components (i) and (ii) may be added together to form an isolable metal-ligand complex and then added to component (iii) and contacted with ethylene, or components (i), (ii) and (iii) may be added together to form an isolable metal-ligand complex and then contacted with ethylene. The solvent used in the oligomerization is preferably the same non-aromatic solvent used in the preparation of the activator (especially hexene, octene, or a mixture of the two).

The catalyst components (i), (ii) and (iii) utilized in the present invention can be unsupported or supported on a support material, for example, silica, alumina, $MgCl_2$ or zirconia, or on a polymer, for example polyethylene, polypropylene, polystyrene, or poly(aminostyrene). It is preferred to use the catalyst in unsupported form. If desired the catalysts can be formed in situ in the presence of the support material or the support material can be pre-impregnated or premixed, simultaneously or sequentially, with one or more of the catalyst components. The quantity of support material employed can vary widely, for example from 100,000 to 1 grams per gram of metal present in the transition metal compound.

The oligomerization can be, conducted under solution phase, slurry phase, gas phase or bulk phase conditions. Suitable temperatures range from 10° C to +300°C preferably from 10° C to 100°C, especially from 20 to 70°C. Suitable pressures are from atmospheric to 81060 kPa (800 atmospheres (gauge)) preferably from 507 to 10,133 kPa (5 atmospheres to 100 atmospheres), especially from 1013 to 5066 kPa (10 to 50 atmospheres).

Irrespective of the process conditions employed, the oligomerization is typically carried out under conditions that substantially exclude oxygen, water, and other materials that act as catalyst poisons. Also, oligomerization can be carried out in the presence of additives to control selectivity, enhance activity and reduce the amount of polymer formed in oligomerization processes. Potentially suitable additives include, but are not limited to, hydrogen or a halide source - with the use of hydrogen being especially preferred.

There exist a number of options for the oligomerization reactor including batch, semi-batch, and continuous operation. The reactions of the present invention can be performed under a range of process conditions that are readily apparent to those skilled in the art: as a homogeneous liquid phase reaction in the presence or absence of an inert hydrocarbon diluent such as mixed heptanes; as a two-phase liquid/liquid reaction; as a slurry process where the catalyst is in a form that displays little or no solubility; as a bulk process in which essentially neat reactant and/or product olefins serve as the dominant medium; as a gas-phase process in which at least a portion of the reactant or product olefin(s) are transported to or from a supported form of the catalyst via the gaseous state. Evaporative cooling from one or more monomers or inert volatile liquids is but one method that can be employed to effect the removal of heat from the reaction. The reactions may be performed in the known types of gas-phase reactors, such as circulating bed, vertically or horizontally stirred-bed, fixed-bed, or fluidized-bed reactors, liquid-phase reactors, such as plug-flow, continuously stirred tank, or loop reactors, or combinations thereof. A wide range of methods for effecting product, reactant, and catalyst separation and/or purification are known to those skilled in the art and may be employed: distillation, filtration, liquid-liquid separation, slurry settling, extraction, etc. One or more of these methods may be performed separately from the oligomerization reaction or it may be advantageous to integrate at least some with the reaction; a non-limiting example of this would be a process employing catalytic (or reactive) distillation. Also advantageous may be a process which includes more than one reactor, a catalyst kill system between reactors or after the final reactor, or an integrated reactor/separator/purifier. While all catalyst components, reactants, inerts, and products could be employed in the present invention on a once-through basis, it is often economically advantageous to recycle one or more of these materials; in the case of the catalyst system, this might require reconstituting one or more of the catalysts components to achieve the active catalyst system. It is within the scope of this invention that an oligomerization product might also serve as a solvent or diluent. Mixtures of inert diluents or solvents also could be employed. The preferred diluents or solvents are aliphatic and olefinic hydrocarbons and halogenated hydrocarbons such as, for example, isobutane, pentane, heptane, cyclohexane, methylcyclohexane, 1-hexene, 1-octene, chlorobenzene, dichlorobenzene, and the like, and mixtures such as Isopar™.

Techniques for varying the distribution of products from the oligomerization reactions include controlling process conditions (e.g. concentration of components (i)-(iii), reaction temperature, pressure, residence time) and properly selecting the design of the process and are well known to those skilled in the art.

The ethylene feedstock for the oligomerization may be substantially pure or may contain other olefinic impurities and/or ethane. One embodiment of the process of the invention comprises the oligomerization of ethylene-containing waste streams from other chemical processes or a crude ethylene/ethane mixture from a cracker.

In a highly preferred embodiment of the present invention, the oligomerization product produced from this invention is added to a product stream from another alpha olefins manufacturing process for separation into different alpha olefins. As previously discussed, "conventional alpha olefin plants" (wherein the term includes i) those processes which produce alpha olefins by a chain growth process using an aluminum alkyl catalyst, ii) the aforementioned "SHOP" process and iii) the production of olefins from synthesis gas using the so called Lurgi process) have a series of distillation columns to separate the "crude alpha product" (i.e. a mixture of alpha olefins) into alpha olefins (such as butene-1, hexene-1 and octene-1). The mixed oligomer product which is produced in accordance with the present invention is highly suitable for addition/mixing with a crude alpha olefin product from an existing alpha olefin plant (or a "cut" or fraction of the product from such a plant) because the mixed hexene-octene product produced in accordance with the present invention can have very low levels of internal olefins. Thus, the oligomer product of the present invention can be readily separated in the existing distillation columns of alpha olefin plants (without causing the large burden on the operation of these distillation columns which would otherwise exist if the present hexene-octene product stream contained large quantities of internal olefins). As used herein, the term "liquid product" is meant to refer to the oligomers produced by the process of the present invention which have from 4 to (about) 20 carbon atoms.

The liquid product from the oligomerization process of the present invention preferably consists of from 20 to 80 weight% octenes (especially from 35 to 75 weight%) octenes and from 15 to 50 weight% (especially from 20 to 40 weight%) hexenes (where all of the weight% are calculated on the basis of the liquid product by 100%. This product may be prepared when using a ligand in which each of $R^1$ to $R^4$ is a phenyl group having an ortho fluro substituent and the bridge is a nitrogen atom having an isopropyl substituent (as shown in the examples).

One embodiment of the present invention encompasses the use of components (i) (ii) and (iii) in conjunction with one or more types of olefin polymerization catalyst system (iv) to trimerise ethylene and subsequently incorporate a portion of the trimerisation product(s) into a higher polymer.

Component (iv) may be one or more suitable polymerization catalyst system(s), examples of which include, but are not limited to, conventional Ziegler-Natta catalysts, metallocene catalysts, monocyclopentadienyl or "constrained geometry" catalysts, phosphinimine catalysts, heat activated supported chromium oxide catalysts (e.g. "Phillips"-type catalysts), late transition metal polymerization catalysts (e.g. diimine, diphosphine and salicylaldimine nickel/palladium catalysts, iron and cobalt pyridyldiimine catalysts and the like) and other so-called "single site catalysts" (SSC's).

Ziegler-Natta catalysts, in general, consist of two main components. One component is an alkyl or hydride of a Group I to III metal, most commonly $Al(Et)_3$ or $Al(iBu)_3$ or $Al(Et)_2Cl$ but also encompassing Grignard reagents, n-butyllithium, or dialkylzinc compounds. The second component is a salt of a Group IV to VIII transition metal, most commonly halides of titanium or vanadium such as $TiCl_4$, $TiCl_3$, $VCl_4$, or $VOCl_3$. The catalyst components when mixed, usually in a hydrocarbon solvent, may form a homogeneous or heterogeneous product. Such catalysts may be impregnated on a support, if desired, by means known to those skilled in the art and so used in any of the major processes known for co-ordination catalysis of polyolefins such as solution, slurry, and gas-phase. In addition to the two major components described above, amounts of other compounds (typically electron donors) maybe added to further modify the polymerization behaviour or activity of the catalyst.

Metallocene catalysts, in general, consist of transition metal complexes, most commonly based on Group IV metals, ligated with cyclopentadienyl(Cp)-type groups. A wide range of structures of this type of catalysts is known, including those with substituted, linked and/or heteroatom-containing Cp groups, Cp groups fused to other ring systems and the like. Additional activators, such as boranes or alumoxane, are often used and the catalysts may be supported, if desired.

Monocyclopentadienyl or "constrained geometry" catalysts, in general, consist of a transition metal complexes, most commonly based on Group IV metals, ligated with one cyclopentadienyl(Cp)-type group, often linked to additional donor group. A wide range of structures of this type of catalyst is known, including those with substituted, linked and/or heteroatom-containing Cp groups, Cp groups fused to other ring systems and a range of linked and non-linked additional donor groups such as amides, amines and alkoxides. Additional activators, such as boranes or alumoxane, are often used and the catalysts may be supported, if desired.

A typical heat activated chromium oxide (Phillips) type catalyst employs a combination of a support material to which has first been added a chromium-containing material wherein at least part of the chromium is in the hexavalent state by heating in the presence of molecular oxygen. The support is generally composed of about 80 to 100 wt. % silica, the remainder, if any, being selected from the group consisting of refractory metal oxides, such as aluminium, boria, magnesia, thoria, zirconia, titania and mixtures of two or more of these refractory metal oxides. Supports can also comprise alumina, aluminium phosphate, boron phosphate and mixtures thereof with each other or with silica. The chromium compound is typically added to the support as a chromium (III) compound such as the acetate or acetylacetonate in order to avoid the toxicity of chromium (VI). The raw catalyst is then calcined in air at a temperature between 250 and 1000°C for a period of from a few seconds to several hours. This converts at least part of the chromium to the hexavalent state. Reduction of the Cr (VI) to its active form normally occurs in the polymerization reaction, but can be done at the end of the calcination cycle with CO at about 350°C. Additional compounds, such as fluorine, aluminium and/or titanium may be added to the raw Phillips catalyst to modify it.

Late transition metal and single site catalysts cover a wide range of catalyst structures based on metals across the transition series.

Component (iv) may also comprise one or more polymerization catalysts or catalyst systems together with one or more additional oligomerization catalysts or catalyst systems. Suitable oligomerization catalysts include, but are not limited to, those that dimerise (for example, nickel phosphine dimerisation catalysts) or trimerise olefins or otherwise oligomerize olefins to, for example, a broader distribution of 1-olefins (for example, iron and cobalt pyridyldiimine oligomerization catalysts).

Component (iv) may independently be supported or unsupported. Where components (i) and (ii) and optionally (iii) are supported, (iv) may be co-supported sequentially in any order or simultaneously on the same support or may be on a separate support. For some combinations, the components (i) (iii) may be part or all of component (iv). For example, if component (iv) is a heat activated chromium oxide catalyst then this may be (i), a chromium source and if component (iv) contains an alumoxane activator then this may also be the optional activator (iii).

The components (i), (ii), (iii) and (iv) may be in essentially any molar ratio that produces a polymer product. The precise ratio required depends on the relative reactivity of the components and also on the desired properties of the product or catalyst systems.

An "in series" process could be conducted by first conducting the oligomerization reaction, then passing the oligomerization product to a polymerization reaction. In the case of an "in series" process various purification, analysis and control steps for the oligomeric product could potentially be incorporated between the trimerization and subsequent reaction stages. Recycling between reactors configured in series is also possible. An example of such a process would be the oligomerization of ethylene in a single reactor with a catalyst comprising components (i)-(iii) followed by copolymerization of the oligomerization product with ethylene in a separate, linked reactor to give branched polyethylene. Another example would be the oligomerization of an ethylene-containing waste stream from a polyethylene process, followed by introduction of the oligomerization product back into the polyethylene process as a co-monomer for the production of branched polyethylene.

An example of an "in situ" process is the production of branched polyethylene catalyzed by components (i)-(iv), added in any order such that the active catalytic species derived from components (i)-(iii) are at some point present in a reactor with component (iv).

Both the "in series and "in situ" approaches can be adaptions of current polymerization technology for the process stages including component (iv). All major olefin existing polymerization processes, including multiple reactor processes, are considered adaptable to this approach. One adaption is the incorporation of an oligomerization catalyst bed into a recycle loop of a gas phase polymerization process, this could be as a side or recycle stream within the main fluidization recycle loop and or within the degassing recovery and recycle system.

Polymerization conditions when component (iv) is present can be, for example, solution phase, slurry phase, gas phase or bulk phase, with temperatures ranging from -100° C to +300°C and at pressures of atmospheric and above, particularly from 1.5 to 50 atmospheres. Reaction conditions, will typically have a significant impact upon the properties (e.g. density, melt index, yield) of the polymer being made and it is likely that the polymer requirements will dictate many of the reaction variables. Reaction temperature, particularly in processes where it is important to operate below the sintering temperature of the polymer, will typically, and preferably, be primarily selected to optimize the polymerization reaction conditions. Also, polymerization or copolymerization can be carried out in the presence of additives to control polymer or copolymer molecular weights. The use of hydrogen gas as a means of controlling the average molecular weight of the polymer or copolymer applies generally to the polymerization process of the present invention.

Slurry phase polymerization conditions or gas phase polymerization conditions are particularly useful for the production of high or low density grades of polyethylene, and polypropylene. In these processes the polymerization conditions can be batch, continuous or semi-continuous. Furthermore, one or more reactors may be used, e.g. from two to five reactors in series. Different reaction conditions, such as different temperatures or hydrogen concentrations may be employed in the different reactors.

Once the polymer product is discharged from the reactor, any associated and absorbed hydrocarbons are substantially removed, or degassed, from the polymer by, for example, pressure let-down or gas purging using fresh or recycled steam, nitrogen or light hydrocarbons (such as ethylene). Recovered gaseous or liquid hydrocarbons may be recycled to a purification system or the polymerization zone.

In the slurry phase polymerization process the polymerization diluent is compatible with the polymer(s) and catalysts, and may be an alkane such as hexane, heptane, isobutane, or a mixture of hydrocarbons or paraffins. The polymerization zone can be, for example, an autoclave or similar reaction vessel, or a continuous liquid full loop reactor, e.g. of the type well-known in the manufacture of polyethylene by the Phillips Process. When the polymerization process of the present invention is carried out under slurry conditions the polymerization is preferably carried out at a temperature above 0°C, most preferably above 15°C. Under slurry conditions the polymerization temperature is preferably maintained below the temperature at which the polymer commences to soften or sinter in the presence of the polymerization diluent. If the temperature is allowed to go above the latter temperature, fouling of the reactor can occur. Adjustment of the polymerization within these defined temperature ranges can provide a useful means of controlling the average molecular weight of the produced polymer. A further useful means of controlling the molecular weight is to conduct the polymerization in the presence of hydrogen gas which acts as chain transfer agent. Generally, the higher the concentration of hydrogen employed, the lower the average molecular weight of the produced polymer.

In bulk polymerization processes, liquid monomer such as propylene is used as the polymerization medium.

Methods for operating gas phase polymerization processes are well known in the art. Such methods generally involve agitating (e.g. by stirring, vibrating or fluidizing) a bed of catalyst, or a bed of the target polymer (i.e. polymer having the same or similar physical properties to that which it is desired to make in the polymerization process) containing a catalyst, and feeding thereto a stream of monomer (under conditions such that at least part of the monomer polymerizes in contact with the catalyst in the bed. The bed is generally cooled by the addition of cool gas (e.g. recycled gaseous monomer) and/or volatile liquid (e.g. a volatile inert hydrocarbon, or gaseous monomer which has been condensed to form a liquid). The polymer produced in, and isolated from, gas phase processes forms directly a solid in the polymerization zone and is free from, or substantially free from liquid. As is well known to those skilled in the art, if any liquid is allowed to enter the polymerization zone of a gas phase polymerization process the quantity of liquid in the polymerization zone is small in relation to the quantity of polymer present. This is in contrast to "solution phase" processes wherein the polymer is formed dissolved in a solvent, and "slurry phase" processes wherein the polymer forms as a suspension in a liquid diluent.

The gas phase process can be operated under batch, semi-batch, or so-called "continuous" conditions. It is preferred to operate under conditions such that monomer is continuously recycled to an agitated polymerization zone containing polymerization catalyst, make-up monomer being provided to replace polymerized monomer, and continuously or intermittently withdrawing produced polymer from the polymerization zone at a rate comparable to the rate of formation of the polymer, fresh catalyst being added to the polymerization zone to replace the catalyst withdrawn from the polymerization zone with the produced polymer.

Methods for operating gas phase fluidized bed processes for making polyethylene, ethylene copolymers and polypropylene are well known in the art. The process can be operated, for example, in a vertical cylindrical reactor equipped with a perforated distribution plate to support the bed and to distribute the incoming fluidizing gas stream through the bed. The fluidizing gas circulating through the bed serves to remove the heat of polymerization from the bed and to supply monomer for polymerization in the bed. Thus the fluidizing gas generally comprises the monomer(s) normally together with some inert gas (e.g. nitrogen or inert hydrocarbons such as methane, ethane, propane, butane, pentane or hexane) and optionally with hydrogen as molecular weight modifier. The hot fluidizing gas emerging from the top of the bed is led optionally through a velocity reduction zone (this can be a cylindrical portion of the reactor having a wider diameter) and, if desired, a cyclone and or filters to disentrain fine solid particles from the gas stream. The hot gas is then led to a heat exchanger to remove at least part of the heat of polymerization. Catalysts are preferably fed continuously or at regular intervals to the bed. At start up of the process, the bed comprises fluidizable polymer which is preferably similar to the target polymer. Polymer is produced continuously within the bed by the polymerization of the monomer(s). Preferably means are provided to discharge polymer from the bed continuously or at regular intervals to maintain the fluidized bed at the desired height. The process is generally operated at relatively low pressure, for example, at 10 to 50 atmospheres, and at temperatures for example, between 50 and 135°C. The temperature of the bed is maintained below the sintering temperature of the fluidized polymer to avoid problems of agglomeration.

In the gas phase fluidized bed process for polymerization of olefins the heat evolved by the exothermic polymerization reaction is normally removed from the polymerization zone (i.e. the fluidized bed) by means of the fluidizing gas stream as described above. The hot reactor gas emerging from the top of the bed is led through one or more heat exchangers wherein the gas is cooled. The cooled reactor gas, together with any make-up gas, is then recycled to the base of the bed. In the gas phase fluidized bed polymerization process of the present invention it is desirable to provide additional cooling of the bed (and thereby improve the space time yield of the process) by feeding a volatile liquid to the bed under conditions such that the liquid evaporates in the bed thereby absorbing additional heat of polymerization from the bed by the "latent heat of evaporation" effect. When the hot recycle gas from the bed enters the heat exchanger, the volatile liquid can condense out. In one embodiment of the present invention the volatile liquid is separated from the recycle gas and reintroduced separately into the bed. Thus, for example, the volatile liquid can be separated and sprayed into the bed. In another embodiment of the present invention the volatile liquid is recycled to the bed with the recycle gas. Thus the volatile liquid can be condensed from the fluidizing gas stream emerging from the reactor and can be recycled to the bed with recycle gas, or can be separated from the recycle gas and then returned to the bed.

A number of process options can be envisaged when using the catalysts of the present invention in an integrated process to prepare higher polymers i.e. when component (iv) is present. These options include "in series" processes in which the oligomerization and subsequent polymerization are carried in separate but linked reactors and "in situ" processes in which a both reaction steps are carried out in the same reactor.

In the case of a gas phase "in situ" polymerization process, component (iv) can, for example, be introduced into the polymerization reaction zone in liquid form, for example, as a solution in a substantially inert liquid diluent. Components (i)-(iv) may be independently added to any part of the polymerization reactor simultaneously or sequentially together or separately. Under these circumstances it is preferred the liquid containing the component(s) is sprayed as fine droplets into the polymerization zone. The droplet diameter is preferably within the range 1 to 1000 microns.

Although not usually required, upon completion of polymerization or copolymerization, or when it is desired to terminate polymerization or copolymerization or at least temporarily deactivate the catalyst or catalyst component of this invention, the catalyst can be contacted with water, alcohols, acetone, or other suitable catalyst deactivators a manner known to persons of skill in the art.

A range of polyethylene polymers are considered accessible including high density polyethylene, medium density polyethylene, low density polyethylene, ultra low density polyethylene and elastomeric materials. Particularly important are the polymers having a density in the range of 0.91 to 0.93, grams per cubic centimeter (g/cc) generally referred to in the art as linear low density polyethylene. Such polymers and copolymers are used extensively in the manufacture of flexible blown or cast film.

Depending upon the use of the polymer product, minor amounts of additives are typically incorporated into the polymer formulation such as acid scavengers, antioxidants, stabilizers, and the like. Generally, these additives are incorporated at levels of about 25 to 2000 parts per million by weight (ppm), typically from about 50 to about 1000 ppm, and more typically 400 to 1000 ppm, based on the polymer. In use, polymers or copolymers made according to the invention in the form of a powder are conventionally compounded into pellets. Examples of uses for polymer compositions made according to the invention include use to form fibers, extruded films, tapes, spunbonded webs, molded or thermoformed products, and the like. The polymers may be blown or cast into films, or may be used for making a variety of molded or extruded articles such as pipes, and containers such as bottles or drums. Specific additive packages for each application may be selected as known in the art. Examples of supplemental additives include slip agents, anti-blocks, anti-stats, mould release agents, primary and secondary anti-oxidants, clarifiers, nucleants, uv stabilizers, and the like. Classes of additives are well known in the art and include phosphite antioxidants, hydroxylamine (such as N,N-dialkyl hydroxylamine) and amine oxide (such as dialkyl methyl amine oxide) antioxidants, hindered amine light (uv) stabilizers, phenolic stabilizers, benzofuranone stabilizers, and the like.

Fillers such as silica, glass fibers, talc, and the like, nucleating agents, and colourants also may be added to the polymer compositions as known by the art. The present invention is illustrated in more detail by the following non-limiting examples.

## EXAMPLES

Comparative Examples

The comparative experiments of this example were conducted using purchased aluminoxanes.

A solution of "pure" MAO (i.e. reported to contain TMA as the only aluminum alkyl) was purchased from Akzo Nobel. This MAO is reported to contain about 10 weight % methylaluminoxane in toluene.

A solution of "modified" MAO or "MMAO" (reported to contain about 70 mole % methyl groups and 30 mole % i-butyl groups) in an aliphatic solvent was purchased from Akzo Nobel under the trade name MMAO-3.

These aluminoxanes were used in a wide variety of oligomerization reactions conducted in a continuous oligomerization reactor. One typical operating condition is described below.

A 1 L reactor fitted with a stirrer (1000 rpm) was purged 3 times with nitrogen while heated at 80°C. The reactor was then filled with a solution of aluminoxane (64 mmol/L at feed rate at 2.50mL/min), and an 1-octene solution of Cr(acac)$_3$ and N,N-bis-[di(2-fluorophenyl)phosphine] isopropylamine (0.213 mmol/L at feed rate of 2.50 mL/min) while maintaining a pressure of 8 MPa. Once the reactor is full, a solution of ethylene (fed at 3 g/min) and hydrogen (fed at 50 sccm) in 1-octene was added to makeup a total flow rate of 33.3 mL/min to the reactor. An outlet valve from the reactor was opened to allow for continuous flow of the product mixture out of the reactor. This product stream was quenched in-line with isopropanol and liquid samples were collected at 30 minute intervals for analysis by GC-FID. The experiment was terminated by stopping the flow of the reactants to the reactor, adding isopropanol to the reactor to immediately quench the contents and flush the reactor with solvent. The reactor was then depressurized and opened. The weight of any solids recovered was recorded. Under these conditions, the amount of polymer formed when using MMAO-3 was observed to be in excess of 10 weight % (of the ethylene consumed) and the amount of polymer formed with MAO was less than 1 weight %.

The "absolute" amounts of polymer formed can be increased by eliminating hydrogen flow.

The "absolute" amounts of polymer formed was observed to be reduced by maintaining good temperature control.

However, relatively larger amounts of polymer formation were observed over a range of conditions when MMAO-3 was used instead of MAO.

Part II - Inventive

*General Experimental*

All reactions were conducted under nitrogen using standard Schlenk techniques or in an inert atmosphere glovebox. 1-octene was purified using known techniques and then stored over activated molecular sieves in an inert atmosphere glovebox. Trimethylaluminum was purchased from Aldrich and used as is.

*Activator 1 (MAO-1)*

To a stirred solution of TMA (0.60 g (0.8 mL), 8.3 mmol) in 1-octene (20 mL) was added water (0.15 mL, 8.3 mmol) over 30 minutes at room temperature. White precipitate formed immediately upon addition of the first drop of water. The reaction mixture was stirred for 2 hours after adding completed.

*Activator 2 (MAO-2)*

To a stirred solution of TMA (0.60 g (0.8 mL), 8.3 mmol) in 1-octene (20 mL) cooled to -65°C was added water (0.15 mL, 8.3 mmol) over 15 min. White precipitate formed immediately upon addition of the first drop of water. The reaction mixture was stirred for a further 2 hours at this temperature, then cooling was removed, the mixture allowed to warm to room temperature and stir for an additional 2 hours.

*Oligomerization Experiments*

The oligomerization examples 1 and 2 (below) were completed the day after the MAO-1 was prepared (as described above).

**Example 1**

A 600-mL reactor fitted with a stirrer (1700 rpm) was purged 3 times with argon while heated at 80 °C. The reactor was then cooled to 30°C and a solution of in situ MAO [MAO-1] (4.40 g, 3.26 wt% MAO) in 64.3.0 g 1-octene, followed by 59.8 g of 1-octene was transferred via a stainless steel cannula to the reactor. The reactor was then pressurized with ethylene (40 barg) and the temperature adjusted to 60°C. A 1-octene solution (14.3 g) of N,N-bis-[di(2-fluorophenyl)phosphine] isopropylamine (4.22 mg, 0.00824 mmol) and chromium acetylacetonate (2.88 mg, 0.00824 mmol) was transferred under ethylene to the pressurized reactor. Immediately after, additional ethylene was added to increase the reactor pressure to 45 bar. The reaction was terminated by stopping the flow of ethylene to the reactor and cooling the contents to 30°C, at which point excess ethylene was slowly released from the reactor cooling the contents to 7°C. The product mixture was transferred to a pre-weighed flask. A sample of the liquid product was analyzed by GC-FID. The solid products were collected, weighed and dried at ambient temperature. The mass of product produced was taken as the difference in weights before and after the reactor contents were added to the flask with the ethanol.

**Example 2**

A 600-mL reactor fitted with a stirrer (1700 rpm) was purged 3 times with argon while heated at 80°C. The reactor was then cooled to 30°C and a solution of in situ MAO [MAO-1] (4.40 g, 3.26 wt% MAO) in 64.4 g 1-octene, followed by 59.5 g of 1-octene was transferred via a stainless steel cannula to the reactor. The reactor was then pressurized with ethylene (40 bar) and the temperature adjusted to 60°C. A 1-octene solution (14.2 g) of N,N-bis-[di(2-fluorophenyl)phosphine] isopropylamine (4.22 mg, 0.00824 mmol) and chromium acetylacetonate (2.88 mg, 0.00824 mmol) was transferred under ethylene to the pressurized reactor. Immediately after, additional ethylene was added to increase the reactor pressure to 45 bar. The reaction was terminated by stopping the flow of ethylene to the reactor and cooling the contents to 30°C, at which point excess ethylene was slowly released from the reactor cooling the contents to 7°C. The product mixture was transferred to a pre-weighed flask. A sample of the liquid product was analyzed by GC-FID. The solid products were collected, weighed and dried at ambient temperature. The mass of product produced was taken as the difference in weights before and after the reactor contents were added to the flask with the ethanol.

TABLE 1

| RUN # | Amount Cr (acac)$_3$ mmol | Ligand : Cr | Al : Cr | Rx Time (min) | Activity (g Product/g Cr/hr) | Total Product Wt (g) | Wt % PE | Liquid Product Distribution / Wt % | | | | | | | |
|---|---|---|---|---|---|---|---|---|---|---|---|---|---|---|
| | | | | | | | | C6's | | | C8's | | | C10+ |
| | | | | | | | | tot. C6's | 1-C6 | 1-C6 selectivity | tot. C8's | 1-C8 | 1-C8 selectivity | |
| 1 | 8.24 | 1 : 1 | 300 : 1 | 20 | 265,937 | 38 | 4.05 | 26.71 | 26.70 | 99.94 | 56.53 | 55.48 | 98.14 | 16.76 |
| 2 | 8.24 | 1 : 1 | 300 : 1 | 20 | 162,361 | 23.2 | 8.74 | 27.42 | 27.42 | 100.00 | 56.00 | 54.96 | 98.14 | 16.58 |

General conditions: Solvent = 1-octene with 7wt% cyclohexane; Pressure = 45 bars; Temperature = 60 °C; Stirrer speed = 1700 rpm; iPrN(P(2-F-C6H4)2)2 & Cr(acac)$_3$ = 8.24 μmol and were added to the reactor pressurized with ethylene; Reaction was quenched with ethanol outside reactor.

**Claims**

1. A process for the oligomerization of ethylene, said process comprising:

A) a first step wherein an activator is prepared by the partial hydrolysis of a solution of an alkyl aluminum with water;
B) a second step comprising contacting:

a) said activator;
b) a catalyst comprising a source of chromium and a ligand defined by the formula $(R^1)(R^2)$-$P^1$-bridge-$P^2(R^3)(R^4)$ wherein $R^1$, $R^2$, $R^3$ and $R^4$ are independently selected from the group consisting of hydrocarbyl and heterohydrocarbyl and the bridge is a divalent moiety that is bonded to both phosphorus atoms; and
c) ethylene,

wherein said a), b) and c) are contacted in an oligomerization reactor under oligomerization conditions, with the provisos that:

1) said first step is conducted in a non aromatic solvent for said alkyl aluminum;
2) said alkyl aluminum consists of trimethylaluminum;
3) the amount of said aluminum used is said first step is from 0.5 to 3 weight %, based on the combined weight of said aluminum and said non aromatic solvent; and
4) said activator is used in said second step within 7 days from when said activator is prepared.

2. The process according to claim 1 wherein said second step is conducted in a liquid medium.

3. The process according to claim 2 wherein said liquid medium is the same material as said non aromatic solvent.

4. The process according to claim 3 wherein said non aromatic solvent is selected from the group consisting of $C_6$ to $C_{20}$ aliphatic hydrocarbons; $C_6$ to $C_{20}$ olefins and mixtures thereof.

5. The process according to claim 4 wherein said non aromatic solvent is a mixture of hexene and octene.

6. The process according to claim 1 wherein said partial hydrolysis is conducted by contacting liquid water with said trimethylaluminum.

7. The process according to claim 6 wherein a co-solvent for said water is further included and wherein the weight ratio of said co-solvent to said water is from 1/10 to 10/1.

8. The process according to claim 7 wherein said hydrolysis is conducted by contacting said trimethylaluminum with a source of water that is associated with a solid particulate carrier.

9. The process according to claim 8 wherein said solid particulate carrier is selected from the group consisting of metal salts and metal oxides.

10. The process according to claim 1 wherein said oligomerization is conducted under continuous flow conditions.

11. The process according to claim 10 wherein said oligomerization conditions are **characterized by** an operating temperature of from 20 to 70°C and an ethylene pressure of from 1013 to 5066 kPa (10 to 50 atmospheres).

12. The process according to claim 1 wherein said partial hydrolysis is further **characterized by** the use of a water/aluminum molar ratio of from 0.3/1 to 1/1.

13. The process according to claim 1 wherein said chromium and said ligand are provided in a molar ratio of from 1/1 to 1/2.

14. The process according to claim 1 wherein said bridge is defined by the formula -$N(R^5)$- and $R^5$ is isopropyl.

**Patentansprüche**

1. Verfahren zur Oligomerisierung von Ethylen, wobei das Verfahren Folgendes umfasst:

   A) einen ersten Schritt, in dem ein Aktivator mittels partieller Hydrolyse einer Lösung eines Alkylaluminium mit Wasser hergestellt wird;
   B) einen zweiten Schritt, umfassend das Inkontaktbringen:

   a) des Aktivators;
   b) eines Katalysators, der eine Chromquelle und einen Liganden umfasst, der durch die Formel $(R^1)(R^2)$-$P^1$-Brücke-$P^2(R^3)(R^4)$ definiert ist, worin $R^1$, $R^2$, $R^3$ und $R^4$ unabhängig aus der aus Hydrocarbyl und Heterohydrocarbyl bestehenden Gruppe ausgewählt sind und die Brücke eine zweiwertige Gruppierung ist, die an beide Phosphoratome gebunden ist; und
   c) von Ethylen,

   wobei a), b) und c) in einem Oligomerisierungsreaktor unter Oligomerisierungsbedingungen in Kontakt gebracht werden, mit der Maßgabe, dass:

   1) der erste Schritt in einem nichtaromatischen Lösungsmittel für das Alkylaluminium durchgeführt wird;
   2) das Alkylaluminium aus Trimethylaluminium besteht;
   3) die Menge des verwendeten Aluminiums im ersten Schritt von 0,5 bis 3 Gew.-%, bezogen auf das kombinierte Gewicht des Aluminiums und des nichtaromatischen Lösungsmittels beträgt; und
   4) der Aktivator innerhalb von 7 Tagen ab der Herstellung des Aktivators in dem zweiten Schritt eingesetzt wird.

2. Verfahren nach Anspruch 1, wobei der zweite Schritt in einem flüssigen Medium durchgeführt wird.

3. Verfahren nach Anspruch 2, wobei das flüssige Medium dasselbe Material ist wie das nichtaromatische Lösungsmittel.

4. Verfahren nach Anspruch 3, wobei das nichtaromatische Lösungsmittel aus der aus aliphatischen $C_6$-$C_{20}$-Kohlenwasserstoffen, $C_6$-$C_{20}$-Olefinen und Gemischen davon bestehenden Gruppe ausgewählt ist.

5. Verfahren nach Anspruch 4, wobei das nichtaromatische Lösungsmittel ein Gemisch aus Hexen und Octen ist.

6. Verfahren nach Anspruch 1, wobei die partielle Hydrolyse durchgeführt wird, indem flüssiges Wasser mit dem Trimethylaluminium in Kontakt gebracht wird.

7. Verfahren nach Anspruch 6, wobei weiters ein Hilfslösungsmittel für das Wasser umfasst ist und wobei das Gewichtsverhältnis zwischen dem Hilfslösungsmittel und dem Wasser 1/10 bis 10/1 beträgt.

8. Verfahren nach Anspruch 7, wobei die Hydrolyse durchgeführt wird, indem das Trimethylaluminium mit einer Wasserquelle, die mit einem festen teilchenförmigen Träger verbunden ist, in Kontakt gebracht wird.

9. Verfahren nach Anspruch 8, wobei der feste teilchenförmige Träger aus der aus Metallsalzen und Metalloxiden bestehenden Gruppe ausgewählt ist.

10. Verfahren nach Anspruch 1, wobei die Oligomerisierung unter Bedingungen kontinuierlichen Flusses durchgeführt wird.

11. Verfahren nach Anspruch 10, wobei die Oligomerisierungsbedingungen durch eine Betriebstemperatur von 20-70 °C und einem Ethylendruck von 1013 bis 5066 kPa (10 bis 50 Atmosphären) gekennzeichnet sind.

12. Verfahren nach Anspruch 1, wobei die partielle Hydrolyse weiters durch ein Wasser/Aluminium-Molverhältnis von 0,3/1 bis 1/1 gekennzeichnet ist.

13. Verfahren nach Anspruch 1, wobei das Chrom und der Ligand in einem Molverhältnis von 1/1 bis 1/2 bereitgestellt werden.

**14.** Verfahren nach Anspruch 1, wobei die Brücke durch die Formel -N($R^5$)- definiert ist und $R^5$ Isopropyl ist.

**Revendications**

**1.** Procédé pour l'oligomérisation d'éthylène, ledit procédé comprenant :

A) une première étape dans laquelle un activateur est préparé par l'hydrolyse partielle d'une solution d'un alkylaluminium avec de l'eau ;
B) une seconde étape comprenant la mise en contact :

a) dudit activateur ;
b) d'un catalyseur comprenant une source de chrome et un ligand défini par la formule ($R^1$($R^2$)-$P^1$-pont-$P^2$($R^3$)($R^4$), dans lequel $R^1$, $R^2$, $R^3$ et $R^4$ sont choisis indépendamment dans le groupe comprenant hydrocarbyle, et hétérohydrocarbyle, et le pont est un fragment divalent qui est lié aux deux atomes de phosphore ; et
c) d'éthylène,

dans lequel lesdits a), b) et c) sont mis en contact dans un réacteur d'oligomérisation sous des conditions d'oligomérisation, à condition que :

1) ladite première étape est exécutée dans un solvant non aromatique pour ledit alkylaluminium ;
2) ledit alkylaluminium consiste en du triméthylaluminium ;
3) la quantité dudit aluminium utilisée dans ladite première étape est de 0,5 à 3 % en poids, sur la base du poids combiné dudit aluminium et dudit solvant non aromatique ; et
4) ledit activateur est utilisé dans ladite seconde étape dans les 7 jours à partir de la préparation dudit activateur.

**2.** Procédé selon la revendication 1, dans lequel ladite seconde étape est exécutée dans un milieu liquide.

**3.** Procédé selon la revendication 2, dans lequel ledit milieu liquide est le même matériau que ledit solvant non aromatique.

**4.** Procédé selon la revendication 3, dans lequel ledit solvant non aromatique est choisi dans le groupe constitué par des hydrocarbures aliphatiques en $C_6$ à $C_{20}$ ; des oléfines en $C_6$ à $C_{20}$ et des mélanges de ceux-ci.

**5.** Procédé selon la revendication 4, dans lequel ledit solvant non aromatique est un mélange d'hexène et d'octène.

**6.** Procédé selon la revendication 1, dans lequel ladite hydrolyse partielle est exécutée par la mise en contact de d'eau liquide avec ledit triméthylaluminium.

**7.** Procédé selon la revendication 6, dans lequel un co-solvant pour ladite eau est en outre inclus, et dans lequel le rapport en poids du co-solvant sur ladite eau est de 1/10 à 10/1.

**8.** Procédé selon la revendication 7, dans lequel ladite hydrolyse est exécutée par la mise en contact dudit triméthylaluminium avec une source d'eau qui est associée à un support particulaire solide.

**9.** Procédé selon la revendication 8, dans lequel ledit support particulaire solide est choisi dans le groupe constitué par des sels métalliques et des oxydes métalliques.

**10.** Procédé selon la revendication 1, dans lequel ladite oligomérisation est exécutée dans des conditions de flux continu.

**11.** Procédé selon la revendication 10, dans lequel lesdites conditions d'oligomérisation sont **caractérisées par** une température de fonctionnement de 20 à 70°C et une pression d'éthylène de 1013 à 5066 kPa (10 à 50 atmosphères).

**12.** Procédé selon la revendication 1, dans lequel ladite hydrolyse partielle est en outre **caractérisée par** l'utilisation d'un rapport molaire eau/aluminium de 0,3/1 à 1/1.

**13.** Procédé selon la revendication 1, dans lequel ledit chrome et ledit ligand sont fournis dans un rapport molaire de 1/1 à 1/2.

**14.** Procédé selon la revendication 1, dans lequel ledit pont est défini par la formule -N(R$^5$)-, et R$^5$ est isopropyle.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5066631 A

- WO 2010034101 A